# EUROPÄISCHE PATENTSCHRIFT

(11) **EP 1 689 756 B1**
(45) Veröffentlichungstag und Bekanntmachung des Hinweises auf die Patenterteilung: **15.08.2007**
(21) Anmeldenummer: 04790989.0
(22) Anmeldetag: 28.10.2004
(51) Int. Cl.: C07F 1/00

(54) **VERFAHREN ZUR HERSTELLUNG VON SALZEN SCHWACH KOORDINIERENDER ANIOMEN, DERARTIGE SALZE SOWIE DEREN VERWENDUNG**
METHOD FOR THE PRODUCTION OF SALTS OF WEAKLY COORDINATING ANIONS, SALTS THEREOF AND USE THEREOF
PROCEDE POUR PREPARER DES SELS D'ANIONS FAIBLEMENT COORDONNEES, SELS CORRESPONDANTS ET LEUR UTILISATION

(30) Priorität: 04.12.2003 DE 10356768
(43) Veröffentlichungstag der Anmeldung: 16.08.2006
(73) Patentinhaber: Universität Karlsruhe (TH), 76128 Karlsruhe (DE)
(72) Erfinder: KROSSING, Ingo, 1004 Lausanne (CH); GONSIOR, Marcin, 82-300 Elblag (PL); MÜLLER, Lutz, 79106 Feiburg (DE)
(74) Vertreter: Müller-Boré & Partner Patentanwälte
(86) Internationale Anmeldenummer: PCT/EP2004/012220
(87) Internationale Veröffentlichungsnummer: WO 2005/054254

(56) Entgegenhaltungen:
- WO-A1-00/53611
- GONSIOR, MARCIN ET AL: "PX4+, P2X5+, and P5X2+ (X = Br, I) salts of the superweak Al(OR)4- anion [R = C(CF3)3]" CHEMISTRY--A EUROPEAN JOURNAL , 8(19), 4475 -4492 CODEN: CEUJED; ISSN: 0947-6539, 2002, XP002314949 in der Anmeldung erwähnt
- KROSSING, INGO: "Reactions of P4 and I2 with Ag[Al(OC(CF3)3)4]: from elusive polyphosphorus cations to subvalent P3I6+ and phosphorus rich P5I2+" JOURNAL OF THE CHEMICAL SOCIETY, DALTON TRANSACTIONS , (4), 500 -512 CODEN: JCSDAA; ISSN: 1472-7773, 2002, XP002314950 in der Anmeldung erwähnt
- BIHLMEIER, ANGELA ET AL: "From weakly coordinating to non-coordinating anions? A simple preparation of the silver salt of the least coordinating anion and its application to determine the ground state structure of the Ag(.eta.2-P4)2+ cation" CHEMISTRY--A EUROPEAN JOURNAL , 10(20), 5041-5051 CODEN: CEUJED; ISSN: 0947-6539, 2004, XP002314952

## Beschreibung

Die vorliegende Erfindung betrifft ein Verfahren zur Herstellung von Salzen schwach koordinierender Anionen des Typs gemäß einer der nachstehenden Formeln (1), (2) oder (3):

M[F-X(OR^{F})ₘ]_{z} (1)

M[(RO)ₘX-F-X(OR^{F})ₘ]_{z} (2)

M[(^{F}RO)ₘX-F-X(OR^{F})ₙ-F-X(OR^{F})ₘ]_{z} (3),

derartige Salze schwach koordinierender Anionen sowie deren Verwendung.

Schwach koordinierende Anionen sind ein gegenwärtig viel bearbeitetes Fachgebiet, und zwar mit Ausrichtungen auf industrielle sowie Grundlagenanwendungen. Beispiele für deren Anwendung sind der Einsatz als Gegenion für einen kationischen Katalysator zur Verstärkung dessen katalytischer Aktivität, als Elektrolytsalze in Batterien, als Bestandteile in ionischen Flüssigkeiten oder als Leitsalze in der Elektrochemie. Wenn solche schwach koordinierenden Anionen besonders Fluor-reiche organische Reste aufweisen, eignen sich diese auch für die Herstellung von Katalysatoren, welche über eine Fluorphasenextraktion wiedergewonnen werden können.

Die im Stand der Technik bekannten schwach koordinierenden Anionen weisen entweder nachteilige Eigenschaften, wie beispielsweise mangelnde thermische und/oder hydrolytische Stabilität oder unzureichende Leitfähigkeit bzw. zu große koordinative Kräfte, auf oder sind nur in geringen Mengen verfügbar. So ist eine der auf diesem Fachgebiet wohlbekanntesten Anionenklassen, die halogenierten Carboranate, nur nach vielstufiger Synthese in kleinen Mengen zugänglich (Gramm-Bereich). Ferner muss bei der Synthese des häufig verwendeten B(C₆F₅)₄⁻ Anions stark explosives C₆F₅Li als Zwischenprodukt verwendet werden.

Aus WO 00/53611 sind schwach koordinierende Anionen des Typs Al(OR)₄⁻ (OR = polyfluoriertes aliphatisches Alkoxid) bekannt.

Im Zuge weiterer Untersuchungen auf diesem Fachgebiet wurde beschrieben, daß sich in Gegenwart von extrem reaktiven Kationen ein Anion des Typs [(^{F}RO)₃Al-F-Al(OR^{F})₃]⁻ bildet (^{F}RO = fluoriertes Alkoxid) (siehe M. Gonsior, I. Krossing, L. Müller, I. Raabe, M. Jansen, L. van Wüllen, Chem. Eur. J. 2002, 8, 4475; I. Krossing, Dalton Trans. 2002, 500). Dieses fluoridverbrückte Anion [(^{F}RO)₃Al-F-Al(OR^{F})₃]⁻ konnte bislang jedoch nur als Zersetzungsprodukt, beispielsweise bei der Reaktion von P₂l₄ mit Ag[Al(OR^{F})₄], erhalten werden.

Dementsprechend ist derzeit kein effektiver Zugang zu derartigen, für industrielle Anwendungen interessanten Salzen mit schwach koordinierenden Anionen bekannt. Eine Analyse der Parameter des vorstehend angeführten Anions [(^{F}RO)₃Al-F-Al(OR^{F})₃]⁻ sowie quantenchemische Rechnungen zeigen allerdings, daß es der beste bisher bekannte schwach koordinierende Anionentyp ist. Durch die erhöhte Anzahl an peripheren Fluoratomen [beispielsweise 54 Fluoratome für ^{F}R = (F₃C)₃C] ist das entsprechende fluoridverbrückte {[(F₃C)₃CO]₃Al-F-Al[OC(CF₃)₃]₃}⁻-Anion noch schwächer koordinierend und stabiler gegen Ligandenabstraktion als das homoleptische {Al[OC(CF₃)₃]₄}⁻-Anion mit nur 36 peripheren Fluoratomen.

Es ist daher Aufgabe der vorliegenden Erfindung, ein Verfahren zur Herstellung von Salzen schwach koordinierender Anionen bereitzustellen, durch welches derartige Salze einfach und in sehr guter Ausbeute in einem beliebigen Maßstab hergestellt werden können.

Diese Aufgabe wird durch die in den Ansprüchen gekennzeichneten Ausführungsformen gelöst.

Insbesondere wird ein Verfahren zur Herstellung von Salzen schwach koordinierender Anionen gemäß einer der nachstehenden Formeln (1), (2) oder (3) bereitgestellt:

M[F-X(OR^{F})ₘ]_{z} (1)

M[(^{F}RO)ₘX-F-X(OR^{F})ₘ]_{z} (2)

M[(^{F}RO)ₘX-F-X(OR^{F})ₙ-F-X(OR^{F})ₘ]_{z} (3)

worin in einem ersten Schritt eine Elementorganylverbindung XRₘ mit einem teilweise oder vollständig fluorierten Alkohol ^{F}ROH in einem organischen, aprotischen Lösungsmittel umgesetzt wird und anschließend in einem zweiten Schritt die erhaltene Elementalkoxyverbindung X(OR^{F})ₘ mit einem zur Abstraktion eines Fluoridions befähigten Fluoridsalz MyY_{z}, gegebenenfalls unter XFₘ-Katalyse, umgesetzt wird,
wobei X aus der Gruppe, bestehend aus B, Al, Ga, In, P, As und Sb, ausgewählt ist,

M ein einwertiges oder zweiwertiges Kation ist,
m 3 oder 5 ist und
n 2 ist, wenn m 3 ist, bzw.
n 4 ist, wenn m 5 ist,
y 1 oder 2 ist, mit der Maßgabe, daß, wenn y 1 ist, Y ein einwertiges Anion ist, bzw. wenn y 2 ist, Y ein zweiwertiges Anion ist, und
z 1 oder 2 ist, mit der Maßgabe, daß, wenn z 1 ist, M ein einwertiges Kation ist, bzw. wenn z 2 ist, M ein zweiwertiges Kation ist.

In einer bevorzugten Ausführungsform der vorliegenden Erfindung wird ein Verfahren zur Herstellung von Salzen schwach koordinierender Anionen gemäß einer der nachstehenden Formeln (1'), (2') oder (3') bereitgestellt:

M[F-Al(OR^{F})₃]_{z} (1')

M[(^{F}RO)₃Al-F-Al(OR^{F})₃]_{z} (2')

M[(RO)₃Al-F-Al(OR^{F})₂-F-Al(OR^{F})₃]_{z} (3')

worin im ersten Schritt eine Aluminiumtriorganylverbindung AIR₃ mit einem teilweise oder vollständig fluorierten Alkohol ^{F}ROH in einem organischen, aprotischen Lösungsmittel umgesetzt wird und anschließend im zweiten Schritt die erhaltene Aluminiumtrialkoxyverbindung Al(OR^{F})₃ mit einem Tetrafluoroboratsalz M(BF₄)_{z}, gegebenenfalls unter BF₃-Katalyse, umgesetzt wird.

Im erfindungsgemäßen Verfahren kann die Elementalkoxyverbindung X(OR^{F})ₘ bevorzugt eine Aluminiumtrialkoxyverbindung Al(OR^{F})₃, mit dem zur Abstraktion eines Fluoridions befähigten Fluoridsalz MyY_{z}, bevorzugt einem Tetrafluoroboratsalz M(BF₄)_{z}, im zweiten Schritt im Verhältnis 1:1, wenn z 1 ist, oder im Verhältnis 2:1, wenn z 1 oder 2 ist, oder im Verhältnis 4:1, wenn z 2 ist, umgesetzt werden.

Für das vorstehend genannte Umsetzungsverhältnis von 1:1, wenn z 1 ist, oder 2:1, wenn z 2 ist, bilden sich nach dem erfindungsgemäßen Verfahren Salze schwach koordinierender Anionen gemäß der Formel (1) bzw. der Formel (1').

Für das vorstehend genannte Umsetzungsverhältnis von 2:1, wenn z 1 ist, oder 4:1, wenn z 2 ist, bilden sich nach dem erfindungsgemäßen Verfahren in Abhängigkeit von den gewählten Reaktionsbedingungen Salze schwach koordinierender Anionen gemäß den Formeln (2), (3), (2') bzw. (3').

Das im erfindungsgemäßen Verfahren verwendete organische, aprotische Lösungsmittel unterliegt keiner spezifischen Beschränkung, sofern es nichtkoordinierend ist und keine Donoreigenschaften aufweist. Üblicherweise ist das Lösungsmittel im erfindungsgemäßen Verfahren aus der Gruppe, bestehend aus Alkanen, Aromaten und halogenierten Aromaten ausgewählt. Bevorzugt ist das Lösungsmittel aus der Gruppe, bestehend aus Pentan, Hexan, Heptan, Oktan, Benzol, Toluol, Kresol, Chlorbenzol und Trichlorbenzol, ausgewählt.

Im ersten Schritt des erfindungsgemäßen Verfahrens wird eine Elementorganylverbindung XRₘ wie insbesondere eine Aluminiumtriorganylverbindung AlR₃ mit einem teilweise oder vollständig fluorierten Alkohol ^{F}ROH in vorstehend genanntem organischen, aprotischen Lösungsmittel umgesetzt. Der Rest R der Elementorganylverbindung ist dabei erfindungsgemäß bevorzugt aus der Gruppe, bestehend aus Wasserstoff, Methyl, Ethyl, n-Propyl, i-Propyl, n-Butyl, i-Butyl, t-Butyl, Phenyl und Tolyl, ausgewählt.

Der im erfindungsgemäßen Verfahren eingesetzte vorstehend genannte teilweise oder vollständig fluorierte Alkohol ^{F}ROH weist bevorzugt einen Rest ^{F}R auf, der aus der Gruppe, bestehend aus geradkettigen oder verzweigtkettigen, teilweise oder vollständig fluorierten C₁ bis C₁₀ Alkylgruppen, teilweise oder vollständig fluorierten C₆ bis C₂₀ Arylgruppen und teilweise oder vollständig fluorierten C₃ bis C₁₀ Cycloalkylgruppen, ausgewählt ist. Insbesondere bevorzugt sind die perfluorierten Gruppen davon.

Exemplarisch können als solche teilweise oder vollständig fluorierten Alkohole ^{F}ROH die folgenden genannt werden: HO-C(CF₃)₃, HO-C(R¹)(CF₃)₂, HO-C(R¹)(R²)(CF₃) und HO-C(R¹)(R²)(R³), wobei R¹, R² und R³ jeweils unabhängig voneinander für Wasserstoff, geradkettige oder verzweigtkettige, teilweise oder vollständig fluorierte C₁ bis C₁₀ Alkylgruppen, teilweise oder vollständig fluorierte C₆ bis C₂₀ Arylgruppen und teilweise oder vollständig fluorierte C₃ bis C₁₀ Cycloalkylgruppen stehen, mit der Maßgabe, daß im Falle von HO-C(R¹)(R²)(R³) mindestens einer von R¹, R² und R³ ein teilweise oder vollständig fluorierter Rest, wie vorstehend aufgeführt, ist. Besonders bevorzugt ist HO-C(CF₃)₃ oder ein vorstehend genannter Alkohol HO-C(R¹)(R²)(R³), der mindestens einen Rest R¹, R² bzw. R³ aufweist, der eine perfluorierte C₁ bis C₁₀ Alkylgruppe, insbesondere C₁ bis C₆ Alkylgruppe, wie z.B. -C₆F₁₃ ist. Letztere eignen sich besonders im Hinblick auf eine spätere Fluorphasenextraktion von entsprechenden Salzen, die aus dem erfindungsgemäßen Verfahren resultieren.

Im zweiten Schritt des erfindungsgemäßen Verfahrens wird ein zur Abstraktion eines Fluoridions befähigtes Fluoridsalz M_{y}Y_{z} eingesetzt. Dabei ist Y üblicherweise aus der Gruppe, bestehend aus BF₄⁻, PF₆⁻, SiF₆²⁻, SbF₄⁻, SbF₆⁻, AsF₄⁻, AsF₆⁻, SnF₅⁻, SnF₆²⁻, GeF₅⁻ und GeF₆²⁻, ausgewählt. Bevorzugt wird im erfindungsgemäßen Verfahren ein Tetrafluoroboratsalt M(BF₄)_{z} als zur Abstraktion eines Fluoridions befähigtes Fluoridsalz verwendet, worin M aus der Gruppe, bestehend aus Alkalimetall-ionen, In⁺, TI⁺, Ag⁺, Cu⁺, NR'₄⁺, PR'₄⁺, wobei R' jeweils unabhängig voneinander für Wasserstoff, einen geradkettigen oder verzweigtkettigen C₁ bis C₂₀-Alkylrest oder unsubstituierten oder substituierten Arylrest, wie insbesondere Phenyl oder Tolyl, steht, und Imidazolium, ausgewählt ist, wenn z 1 ist, oder M aus der Gruppe der Übergangsmetalle, bevorzugt aus der Gruppe, bestehend aus Ni²⁺, Cu²⁺, Zn²⁺, Pd²⁺, Rh²⁺ und Pt²⁺, ausgewählt ist, wenn z 2 ist.

In einer besonders bevorzugten Ausführungsform des Verfahrens gemäß der vorliegenden Erfindung wird
i) im ersten Schritt die Aluminiumtriorganylverbindung AlMe₃ mit dem teilweise oder vollständig fluorierten Alkohol ^{F}ROH in Pentan im Verhältnis 1:3 umgesetzt und anschließend im zweiten Schritt die erhaltene Aluminiumtrialkoxyverbindung Al(OR^{F})₃ mit einem Tetrafluoroboratsalz M(BF₄)_{z} im Verhältnis 1:1, wenn z 1 ist, oder im Verhältnis 2:1, wenn z 2 ist, unter Erhalten einer Verbindung gemäß der vorstehenden Formel (1') umgesetzt,
ii) im ersten Schritt die Aluminiumtriorganylverbindung AlMe₃ mit dem teilweise oder vollständig fluorierten Alkohol ^{F}ROH in Pentan im Verhältnis 1:3 umgesetzt und anschließend im zweiten Schritt die erhaltene Aluminiumtrialkoxyverbindung Al(OR^{F})₃ mit einem Tetrafluoroboratsalz M(BF₄)_{z} im Verhältnis 2:1, wenn z 1 ist, oder im Verhältnis 4:1, wenn z 2 ist, unter Erhalten einer Verbindung gemäß der vorstehenden Formel (2') umgesetzt, oder
iii) im ersten Schritt die Aluminiumtriorganylverbindung AlMe₃ mit dem teilweise oder vollständig fluorierten Alkohol ^{F}ROH in Heptan im Verhältnis 1:3 umgesetzt und anschließend im zweiten Schritt die erhaltene Aluminiumtrialkoxyverbindung Al(OR^{F})₃ mit einem Tetrafluoroboratsalz M(BF₄)_{z} im Verhältnis 2:1, wenn z 1 ist, oder im Verhältnis 4:1, wenn z 2 ist, unter Erhalten einer Verbindung gemäß der vorstehenden Formel (3') umgesetzt.

In den alternativen Ausführungsformen i) bis iii) des erfindungsgemäßen Verfahrens wird im ersten Schritt besonders bevorzugt ein vollständig fluorierter Alkohol ^{F}ROH eingesetzt, worin R^{F} (F₃C)₃C ist.

Darüberhinaus wird in den vorstehend aufgeführten alternativen Ausführungsformen des erfindungsgemäßen Verfahrens im zweiten Schritt bevorzugt ein Tetrafluoroboratsalz MBF₄ eingesetzt, worin M Ag⁺ oder NBu₄⁺ ist.

Die vorliegende Erfindung betrifft weiter Salze mit einem schwach koordinierenden Anion, dargestellt durch die Formel (3):

M[(^{F}RO)ₘX-F-X(OR^{F})ₙ-F-X(OR^{F})ₘ]_{z} (3)

worin X aus der Gruppe, bestehend aus B, Al, Ga, In, P, As und Sb, ausgewählt ist,
M ein einwertiges oder zweiwertiges Kation ist,
m 3 oder 5 ist und
n 2 ist, wenn m 3 ist, bzw.
n 4 ist, wenn m 5 ist,
z 1 oder 2 ist, mit der Maßgabe, daß, wenn z 1 ist, M ein einwertiges Kation ist, bzw. wenn z 2 ist, M ein zweiwertiges Kation ist, und
worin M aus der Gruppe, bestehend aus Alkalimetallionen, In⁺, Tl⁺, Ag⁺, Cu⁺, NR'₄⁺, PR'₄⁺, wobei R' jeweils unabhängig voneinander für Wasserstoff, einen geradkettigen oder verzweigtkettigen C₁ bis C₂₀-Alkylrest oder unsubstituierten oder substituierten Arylrest, wie insbesondere Phenyl oder Tolyl, steht, und Imidazolium, ausgewählt ist, wenn z 1 ist, oder M aus der Gruppe der Übergangsmetalle, bevorzugt aus der Gruppe, bestehend aus Ni²⁺, Cu²⁺, Zn²⁺, Pd²⁺, Rh²⁺ und Pt²⁺, ausgewählt ist, wenn z 2 ist, und

R^{F} aus der Gruppe, bestehend aus geradkettigen oder verzweigtkettigen, teilweise oder vollständig fluorierten C₁ bis C₁₀ Alkylgruppen, teilweise oder vollständig fluorierten C₆ bis C₂₀ Arylgruppen und teilweise oder vollständig fluorierten C₃ bis C₁₀ Cycloalkylgruppen, ausgewählt ist, wobei die perfluorierten Gruppen davon besonders bevorzugt sind.

In einer bevorzugten Ausführungsform betrifft die vorliegende Erfindung Salze mit einem schwach koordinierenden Anion, dargestellt durch die Formel (3'):

M[(^{F}RO)₃Al-F-Al(OR^{F})₂-F-Al(OR^{F})₃]_{z} (3')

worin z, M und R^{F} wie vorstehend definiert sind.

In einer besonders bevorzugten Ausführungsform der erfindungsgemäßen Salze mit der Formel (3) bzw. (3') ist M Ag⁺ oder NBu₄⁺ und R^{F} (F₃C)₃C.

Die erfindungsgemäßen Salze mit schwach koordinierenden Anionen haben ein breites industrielles Anwendungsgebiet. Sie können als Hilfsstoffe in ionischen Flüssigkeiten oder in Li-lonen-Batterien, in der Elektrochemie als inerte Leitsalze in beispielsweise Elektrolyten von Li⁺-lonen oder in der homogenen Katalyse wie z.B. der Olefin-Polymerisation, der Metallocen-Katalyse, der Li⁺-Katalyse von organischen Reaktionen und anderen organischen Synthesen Anwendung finden. Somit betrifft ein weiterer Gegenstand der vorliegenden Erfindung die Verwendung derartiger erfindungsgemäßer Salze insbesondere in ionischen Flüssigkeiten, in Li-Ionen-Batterien, als Leitsalz in der Elektrochemie oder in der homogenen Katalyse.

Die erfindungsgemäßen Anionen weisen gegenüber herkömmlichen Anionen Vorteile hinsichtlich Stabilität, schwacher Koordinationskraft und synthetischer Zugänglichkeit auf.

Darüberhinaus betrifft die vorliegende Erfindung die im erfindungsgemäßen Verfahren zur Herstellung von Salzen schwach koordinierender Anionen als Zwischenprodukte erhaltbaren Elementalkoxyverbindungen, dargestellt durch die Formel (4):

X(OR^{F})ₘ (4)

worin X aus der Gruppe, bestehend aus B, Al, Ga, In, P, As und Sb, ausgewählt ist,
m 3 oder 5 ist und
R^{F} aus der Gruppe, bestehend aus geradkettigen oder verzweigtkettigen, teilweise oder vollständig fluorierten C₁ bis C₁₀-Alkylgruppen, teilweise oder vollständig fluorierten C₆ bis C₂₀-Arylgruppen und teilweise oder vollständig fluorierten C₃ bis C₁₀-Cycloalkylgruppen, ausgewählt ist, wobei die perfluorierten Gruppen davon besonders bevorzugt sind. Exemplarisch können in Formel (4) als solche teilweise oder vollständig fluorierten Alkoxyreste (OR^{F}) wiederum die folgenden genannt werden: O-C(CF₃)₃, O-C(R¹)(CF₃)₂, O-C(R¹)(R²)(CF₃) und O-C(R¹)(R²)(R³), wobei R¹, R² und R³ jeweils unabhängig voneinander für Wasserstoff, geradkettige oder verzweigtkettige, teilweise oder vollständig fluorierte C₁ bis C₁₀ Alkylgruppen, teilweise oder vollständig fluorierte C₆ bis C₂₀ Arylgruppen und teilweise oder vollständig fluorierte C₃ bis C₁₀ Cycloalkylgruppen stehen, mit der Maßgabe, daß im Falle von O-C(R¹)(R²)(R³) mindestens einer von R¹, R² und R³ ein teilweise oder vollständig fluorierter Rest, wie vorstehend aufgeführt, ist. Besonders bevorzugt ist O-C(CF₃)₃ oder ein vorstehend genannter Alkoxyrest O-C(R¹)(R²)(R³), der mindestens einen Rest R¹ R² bzw. R³ aufweist, der eine perfluorierte C₁ bis C₁₀ Alkylgruppe, insbesondere C₁ bis C₆ Alkylgruppe, wie z.B. -C₆F₁₃ ist.

In einer besonders bevorzugten Ausführungsform der erfindungsgemäßen Element-alkoxyverbindungen mit der Formel (4) ist X Al und R^{F} wie vorstehend definiert.

Die vorliegende Erfindung wird durch die nachstehenden, nicht-einschränkenden Beispiele weiter erläutert.

### Beispiele

Aufgrund der Hydrolyse- und Oxidationsempfindlichkeit von AlMe₃ wurden sämtliche Arbeiten unter Ausschluss von Luft und Feuchtigkeit in entsprechenden Apparaturen in einer N₂-Inertgasatmosphäre mit Schlenk- und Handschuhbox-Techniken durchgeführt. Die Glasgeräte waren z.T. mit Hähnen der Fa. J. YOUNG bzw. mit Glasstopfen verschlossen und wurden vor Versuchsbeginn im Ölpumpenvakuum ausgeheizt. Lösungsmittel wurden nach Standardmethoden getrocknet, destilliert, entgast und unter Stickstoff über Molekularsieb (400 pm) aufbewahrt. Zur Bestimmung der bei den Reaktionen entstehenden Gasmengen wurde eine mit Wasser gefüllte, skalierte Glasröhre über einen Schlauch mit der Apparatur verbunden. Durch die Verdrängung des Wassers konnte auf die Gasvolumina geschlossen werden.

### NMR-Spektren

NMR-Spektren wurden unter Verwendung von CD₂Cl₂-Lösungen bei 20°C mittels eines BRUKER AC 250 Spektrometers aufgenommen, als Referenz wurde SiMe₄ (¹H, ¹³C) und AlCl₃ (²⁷Al) verwendet. Die Verschiebungen sind in ppm angegeben.
s = singulett, d = dublett, q = quartett, m = multiplett; J = Kopplungskonstante (Hz)

### HiResESI-Spektren

HiResESI-Spektren wurden unter Verwendung von CH₂Cl₂-Lösungen bei 20°C mittels eines IonSpec Ultima FT-ICR-MS aufgenommen.

In den folgenden Beispielen steht R^{F} für (F₃C)₃C.

### Beispiel 1

### Darstellung von Ag[F-Al(OR^{F})₃]

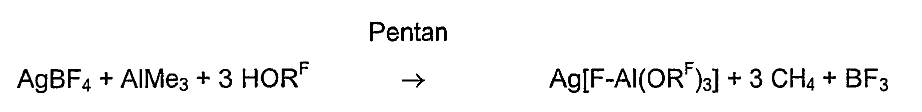

In einem 250 ml Zweihalskolben, der mit einem mittels Kryostaten auf -20°C gekühlten Rückflußkühler ausgestattet ist, werden bei 0°C 1,40 ml (2,82 mmol) Al-Me₃ (gelöst in n-Heptan, c = 2,0 mol/l) in ca. 20 ml Pentan vorgelegt. Anschließend werden 1,18 ml (8,47 mmol) an Alkohol R^{F}OH unter weiterem Rühren zugetropft. Nach vollständiger Methanbildung (190 ml; 100 %) gibt man zu der Mischung aus dem bereits gebildeten Al(OR^{F})₃ auf einmal 0,550 g (2,82 mmol) festes hellbeiges AgBF₄-Salz mittels eines Schlenkgefäßes hinzu. Unter Rühren reagiert das Salz mit dem Al(OR^{F})₃, wobei sich ein hellbeiger Rückstand bildet. Nach der Gasbildung von BF₃ (31 ml; 100 %) wird das Lösungsmittel im Hochvakuum bei 0°C entfernt. Zurück bleibt ein hellgelbes Pulver, welches konstant gewogen wird. Dieses lässt sich dem gewünschten Produkt Ag[F-Al(OR^{F})₃] zuordnen (Ausbeute: 2,375 g; 98%).

**Tab. 1: ¹³C- und ²⁷Al-NMR-Daten der Verbindung Ag[F-Al(OR^{F})₃]**

| Verb. / Fragment | NMR-Kern | δ [ppm] exp. |
|---|---|---|
| OC(**C**F₃)₃ | ¹³C | 119,3 q; ¹J_{CF} = 290,0 Hz |
| O**C**(CF₃)₃ | | 80,6 breit |
| [F-**Al**(OR^{F})₃]⁻ | ²⁷Al | 41 d; ¹J_{AIF} = 67,6 Hz |

Das HiResESI-Spektrum in CH₂Cl₂ bestätigt für das Anion [F-Al(OR^{F})₃]⁻ die theoretische Masse von 751.

### Beispiel 2

### Darstellung von [NBu₄][F-Al(OR^{F})₃]

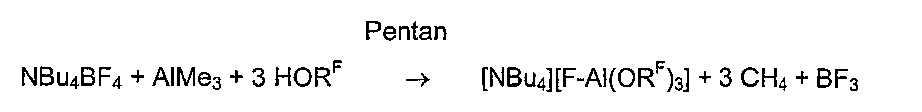

In einem Zweihalskolben, der mit einem mittels Kryostaten auf -20°C gekühlten Rückflußkühler ausgestattet ist, werden ca. 20 ml Pentan und 1,40 ml (2,82 mmol) AlMe₃ (gelöst in n-Heptan, c = 2,0 mol/l) vorgelegt. Bei 0°C werden unter Rühren und Rückfluss 1,18 ml (8,47 mmol) R^{F}OH zugetropft. Nach der vollständigen Methanbildung (190 ml; 100 %) entsteht weißes AL(OR^{F})₃. Nach Zugabe von 0,929 g (2,82 mmol) des weißen NBu₄BF₄-Salzes mittels eines Schlenkgefäßes bildet sich mit der Zeit ein heller Rückstand, der sich absetzt. Die Bildung von BF₃-Gas ist mit 62 ml (100 %) vollständig. Nach Entfernen des Lösungsmittels im Hochvakuum bleibt ein helles festes Pulver zurück, welches konstant gewogen wird und dem Produkt [(NBu₄][F-Al(OR^{F})₃] entspricht (Ausbeute: 2,394 g; 85%).

**Tab. 2: NMR-Daten von [NBu₄][F-Al(OR^{F})₃]**

| Verb. / Fragment | NMR-Kern | δ [ppm] exp. |
|---|---|---|
| N(C₄H₉)₄⁺ | ¹H | 0,95 m + 1,37 m + 1,55 m + 3,05 m |
| OC(CF₃)₃ | ¹³C | 121,6 q; ¹J_{CF} = 291,6 Hz |
| OC(CF₃)₃ | | 79,9 breit |
| N(C₄H₉)₄⁺ | | 13,2 s + 19,6 s + 23,9 s + 58,9 s |
| [F-Al(OR^{F})₃]⁻ | ²⁷Al | 42 s |

Das HiResESI-Spektrum zeigt deutlich die Masse des entsprechenden [F-Al(OR^{F})₃]⁻-Anions bei 751 analog zu der Anionenmasse in der Silbersalzverbindung Ag[F-Al(OR^{F})₃] aus Beispiel 1.

### Beispiel 3

### Darstellung von Ag((R^{F}O)₃Al-F-Al(OR^{F})₃]

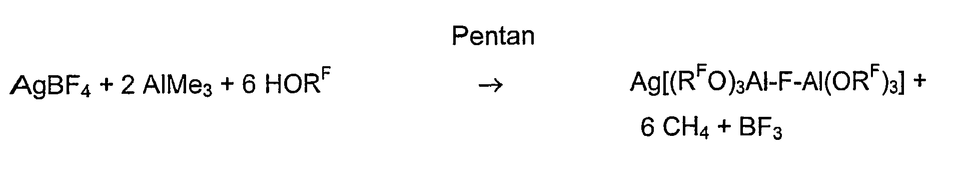

I n einem 250 ml Zweihalskolben, der mit einem mittels Kryostaten auf -20°C gekühlten Rückflußkühler ausgestattet ist, werden bei 0°C unter Rühren 1,40 ml (2,82 mmol) AlMe₃ (gelöst in n-Heptan, c = 2,0 mol/l) in ca. 20 ml Pentan vorgelegt. Während der Zugabe von 1,18 ml (8,47 mmol) R^{F}OH entstehen 190 ml (100 %) an Methan. Nach abgeschlossener Gasentwicklung bildet sich weißes Al(OR^{F})₃. Das AgBF₄-Salz (0,275 g; 1,412 mmol) wird mittels eines Schlenkgefäßes zu dem Gemisch auf einmal zugegeben. Sofort bildet sich ein zäher hellbeiger Feststoff in einer überstehenden farblosen Lösung. Die Menge an entstehendem BF₃-Gas ist vollständig (31 ml; 100%). Nach Abziehen des Lösungsmittels im Hochvakuum bleibt ein beiges grobkörniges Pulver zurück, welches konstant gewogen wird und dem Produkt Ag[(R^{F}O)₃Al-F-Al(ORF)₃] entspricht (Ausbeute: 1,939 g; 86%).

**Tab. 3: NMR-Daten von Ag[(R^{F}O)₃Al-F-Al(OR^{F})₃]**

| Verb. / Fragment | NMR-Kern | δ [ppm] exp. |
|---|---|---|
| OC(CF₃)₃ | ¹³C | 120,9 q; ¹J_{CF} = 291,1 Hz |
| OC(CF₃)₃ | | 79,9 breit |
| [(R^{F}O)₃Al-F-Al(OR^{F})3]⁻ | ²⁷Al | 34 s breit |

### Beispiel 4

### Darstellung von [NBu₄][(R^{F}O)₃Al-F-Al(OR^{F})₃]

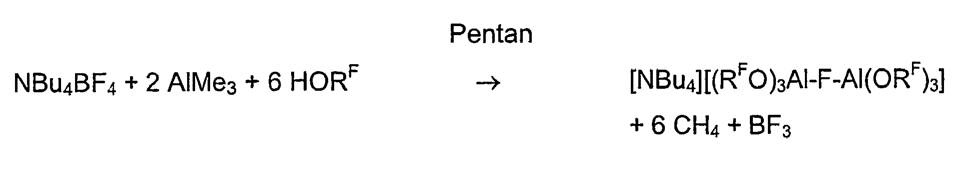

In einem 250 ml Zweihalskolben, der mit einem mittels Kryostaten auf -20°C gekühlten Rückflußkühler ausgestattet ist, werden bei 0°C unter Rühren 1,40 ml (2,82 mmol) AlMe₃ (gelöst in n-Heptan, c = 2,0 mol/l) in ca. 20 ml Pentan vorgelegt. Während der Zugabe von 1,18 ml (8,47 mmol) R^{F}OH entstehen 190 ml (100 %) an Methan. Nach abgeschlossener Gasentwicklung bildet sich weißes Al(OR^{F})₃. Das NBu₄BF₄-Salz (0,464 g; 1,412 mmol) wird mittels eines Schlenkgefäßes zu dem Gemisch auf einmal zugegeben. Sofort bildet sich ein heller Rückstand. Die Menge an entstehendem BF₃-Gas ist vollständig (31 ml; 100%). Nach Abziehen des Lösungsmittels im Hochvakuum bleibt ein farbloser, leicht gelblicher Feststoff zurück, welcher konstant gewogen wird und dem Produkt [NBu₄][(R^{F}O)₃Al-F-Al(OR^{F})₃] entspricht (Ausbeute: 2,17 g; 89%).

**Tab. 4: NMR-Daten von [NBu₄][(R^{F}O)₃Al-F-Al(OR^{F})₃]**

| Verb. / Fragment | NMR-Kern | δ [ppm] exp. |
|---|---|---|
| N(C₄H₉)₄⁺ | ¹H | 0,98 m + 1,37 m + 1,55 m + 3,01 m |
| OC(CF₃)₃ | ¹³C | 120,9 q; ¹J_{CF} = 291,2 Hz |
| OC(CF₃)₃ | | 80 breit |
| N(C₄H₉)₄⁺ | | 13,2 s + 19,8 s + 24,0 s + 59,3 s |
| [(R^{F}O)₃Al-F-Al(OR^{F})₃]⁻ | ²⁷Al | 34 s breit |

### Beispiel 5

### Darstellung von [NBu₄][(R^{F}O)₃Al-F-Al(OR^{F})₂-F-Al(OR^{F})₃]

In einem Zweihalskolben, der mit einem mittels Kryostaten auf -20°C gekühlten Rückflußkühler ausgestattet ist, werden bei 0°C unter Rühren und Rückfluss 1,40 ml (2,82 mmol) AlMe₃ (gelöst in n-Heptan, c = 2,0 mol/l) in ca. 30 ml Heptan vorgelegt. Unter weiterem Rühren gibt man 1,18 ml (8,47 mmol) an R^{F}OH tropfenweise hinzu. Es entsteht weißes Al(OR^{F})₃. Nach vollständiger Methanentwicklung gibt man auf einmal 0,464 g (1,41 mmol) weißes NBu₄BF₄-Salz, gelöst in ca. 5 ml CH₂Cl₂, zu der Gemisch. Es bildet sich eine dunkelgelbe Lösung, in der sich wenig hellbeiger Niederschlag absetzt. Nach vollständiger Entstehung von BF₃-Gas (31 ml; 100 %) lässt man noch ca. eine Stunde bei 0°C weiterrühren. Anschließend wird noch für 2 Stunden refluxiert und das Lösungsmittel im Hochvakuum entfernt. Zurück bleiben 2,453 g eines hellbeigen ölig-festen Rückstands, der aus CH₂Cl₂ umkristallisiert wird. Es ergibt sich das Produkt:
[NBu]₄[(R^{F}O)₃Al-F-Al(OR^{F})₂-F-Al (OR^{F})₃].

**Tab. 5: NMR-Daten von [NBu₄][(R^{F}O)₃Al-F-Al(OR^{F})₂-F-Al(OR^{F})₃]**

| Verb. / Fragment | NMR-Kern | δ [ppm] exp. |
|---|---|---|
| N(C₄H₉)₄⁺ | ¹H | 0,95 m +1,36 m + 1,49 m + 3,03 m |
| N(C₄H₉)₄⁺ | ¹³C | 13,3 s + 19,9 s + 24,1 s + 59,4 s |
| OC(CF₃)₃ | | 120,9 q; ¹J_{CF} = 291,1 Hz¹⁾ |
| | | 121,9 q; ¹J_{CF} = 290,9 HZ²⁾ |
| | | 120,8 q; ¹J_{CF} = 290,9 Hz³⁾ |
| OC(CF₃)₃ | | 79,9 breit |
| [(R^{F}O)₃Al-F-Al(OR^{F})₂-F-Al(OR^{F})₃]⁻ | ²⁷Al | 35 s, sehr breit |

## Patentansprüche

1. Verfahren zur Herstellung von Salzen schwach koordinierender Anionen gemäß einer der nachstehenden Formeln (1), (2) oder (3):
M[F-X(OR^{F})ₘ]_{z} (1)
M[(^{F}RO)ₘX-F-X(OR^{F})ₘ]_{z} (2)
M[(^{F}RO)ₘX-F-X(OR^{F})ₙ-F-X(OR^{F})ₘ]_{z} (3)
worin in einem ersten Schritt eine Elementorganylverbindung XRₘ mit einem teilweise oder vollständig fluorierten Alkohol ^{F}ROH in einem organischen, aprotischen Lösungsmittel umgesetzt wird und anschließend in einem zweiten Schritt die erhaltene Elementalkoxyverbindung X(OR^{F})ₘ mit einem zur Abstraktion eines Fluoridions befähigten Fluoridsalz MyY_{z}, gegebenenfalls unter XFₘ-Katalyse, umgesetzt wird,
wobei X aus der Gruppe, bestehend aus B, Al, Ga, In, P, As und Sb, ausgewählt ist,
M ein einwertiges oder zweiwertiges Kation ist,
m 3 oder 5 ist und
n 2 ist, wenn m 3 ist, bzw.
n 4 ist, wenn m 5 ist,
y 1 oder 2 ist, mit der Maßgabe, daß, wenn y 1 ist, Y ein einwertiges Anion ist, bzw. wenn y 2 ist, Y ein zweiwertiges Anion ist, und
z 1 oder 2 ist, mit der Maßgabe, daß, wenn z 1 ist, M ein einwertiges Kation ist, bzw. wenn z 2 ist, M ein zweiwertiges Kation ist.

2. Verfahren zur Herstellung von Salzen schwach koordinierender Anionen gemäß einer der nachstehenden Formeln (1'), (2') oder (3') nach Anspruch 1:
M[F-Al(OR^{F})₃]_{z} (1')
M[(^{F}RO)₃Al-F-Al(OR^{F})₃]_{z} (2')
M[(^{F}RO)₃Al-F-Al(OR^{F})₂-F-Al(OR^{F})₃]_{z} (3')
worin im ersten Schritt eine Aluminiumtriorganylverbindung AlR₃ mit einem teilweise oder vollständig fluorierten Alkohol ^{F}ROH in einem organischen, aprotischen Lösungsmittel umgesetzt wird und anschließend im zweiten Schritt die erhaltene Aluminiumtrialkoxyverbindung Al(OR^{F})₃ mit einem Tetrafluoroboratsalz M(BF₄)_{z}, gegebenenfalls unter BF₃-Katalyse, umgesetzt wird.

3. Verfahren nach Anspruch 1 oder 2, worin die Aluminiumtrialkoxyverbindung AL(OR^{F})₃ mit dem Tetrafluoroboratsalz M(BF₄)_{z} im Verhältnis 1:1 umgesetzt wird, wenn z 1 ist, oder im Verhältnis 2:1 umgesetzt wird, wenn z 2 ist.

4. Verfahren nach Anspruch 1 oder 2, worin die Aluminiumtrialkoxyverbindung Al(OR^{F})₃ mit dem Tetrafluoroboratsalz M(BF₄)_{z} im Verhältnis 2:1 umgesetzt wird, wenn z 1 ist, oder im Verhältnis 4:1 umgesetzt wird, wenn z 2 ist.

5. Verfahren nach einem der vorhergehenden Ansprüche, worin das organische, aprotische Lösungsmittel aus der Gruppe, bestehend aus Pentan, Hexan, Heptan, Oktan, Benzol, Toluol, Kresol, Chlorbenzol und Trichlorbenzol, ausgewählt ist.

6. Verfahren nach einem der vorhergehenden Ansprüche, worin R ein Rest, ausgewählt aus der Gruppe, bestehend aus Wasserstoff, Methyl, Ethyl, n-Propyl, i-Propyl, n-Butyl, i-Butyl, t-Butyl, Phenyl und Tolyl, ist.

7. Verfahren nach einem der vorhergehenden Ansprüche, worin R^{F} aus der Gruppe, bestehend aus geradkettigen oder verzweigtkettigen, teilweise oder vollständig fluorierten C₁ bis C₁₀-Alkylgruppen, teilweise oder vollständig fluorierten C₆ bis C₂₀-Arylgruppen und teilweise oder vollständig fluorierten C₃ bis C₁₀-Cycloalkylgruppen, ausgewählt ist.

8. Verfahren nach einem der vorhergehenden Ansprüche, worin M aus der Gruppe, bestehend aus Alkalimetallionen, In⁺, TI⁺, Ag⁺, Cu⁺, NR'₄⁺, PR'₄⁺, wobei R' jeweils unabhängig voneinander für Wasserstoff, einen geradkettigen oder verzweigtkettigen C₁ bis C₂₀-Alkylrest oder unsubstituierten oder substituierten Arylrest steht, und Imidazolium, ausgewählt ist, wenn z 1 ist, oder M aus der Gruppe, bestehend aus Ni²⁺, Cu²⁺, Zn²⁺, Pd²⁺, Rh²⁺ und Pt²⁺, ausgewählt ist, wenn z 2 ist.

9. Verfahren nach einem der Ansprüche 1 bis 8, worin im ersten Schritt die Aluminiumtriorganylverbindung AlMe₃ mit dem teilweise oder vollständig fluorierten Alkohol ^{F}ROH in Pentan im Verhältnis 1:3 umgesetzt wird und anschließend im zweiten Schritt die erhaltene Aluminiumtrialkoxyverbindung Al(OR^{F})₃ mit einem Tetrafluoroboratsalz M(BF₄)_{z} im Verhältnis 1:1, wenn z 1 ist, oder im Verhältnis 2:1, wenn z 2 ist, unter Erhalten einer Verbindung gemäß der vorstehenden Formel (1') umgesetzt wird.

10. Verfahren nach einem der Ansprüche 1 bis 8, worin im ersten Schritt die Aluminiumtriorganylverbindung AlMe₃ mit dem teilweise oder vollständig fluorierten Alkohol ^{F}ROH in Pentan im Verhältnis 1:3 umgesetzt wird und anschließend im zweiten Schritt die erhaltene Aluminiumtrialkoxyverbindung Al(OR^{F})₃ mit dem Tetrafluoroboratsalz M(BF₄) im Verhältnis 2:1, wenn z 1 ist, oder im Verhältnis 4:1, wenn z 2 ist, unter Erhalten einer Verbindung gemäß der vorstehenden Formel (2') umgesetzt wird.

11. Verfahren nach einem der Ansprüche 1 bis 8, worin im ersten Schritt die Aluminiumtriorganylverbindung AlMe₃ mit dem teilweise oder vollständig fluorierten Alkohol ^{F}ROH in Heptan im Verhältnis 1:3 umgesetzt wird und anschließend im zweiten Schritt die erhaltene Aluminiumtrialkoxyverbindung Al(OR^{F})₃ mit dem Tetrafluoroboratsalz MBF₄ im Verhältnis 2:1, wenn z 1 ist, oder im Verhältnis 4:1, wenn z 2 ist, unter Erhalten einer Verbindung gemäß der vorstehenden Formel (3') umgesetzt wird.

12. Verfahren nach einem der Ansprüche 9 bis 11, worin R^{F} (F₃C)₃C ist.

13. Verfahren nach einem der Ansprüche 9 bis 12, worin M Ag⁺ oder NBu₄⁺ ist.

14. Salz mit schwach koordinierendem Anion, dargestellt durch die Formel (3):
M[(^{F}RO)ₘX-F-X(OR^{F})ₙ-F-X(OR^{F})ₘ]_{z} (3)
worin X aus der Gruppe, bestehend aus B, Al, Ga, In, P, As und Sb, ausgewählt ist,
M ein einwertiges oder zweiwertiges Kation ist,
m 3 oder 5 ist und
n 2 ist, wenn m 3 ist, bzw.
n 4 ist, wenn m 5 ist,
z 1 oder 2 ist, mit der Maßgabe, daß, wenn z 1 ist, M ein einwertiges Kation ist, bzw. wenn z 2 ist, M ein zweiwertiges Kation ist, und
worin M aus der Gruppe, bestehend aus Alkalimetallionen, In⁺, TI⁺, Ag⁺, Cu⁺, NR'₄⁺, PR'₄⁺, wobei R' jeweils unabhängig voneinander für Wasserstoff, einen geradkettigen oder verzweigtkettigen C₁ bis C₂₀-Alkylrest oder unsubstituierten oder substituierten Arylrest steht, und Imidazolium, ausgewählt ist, wenn z 1 ist, oder M aus der Gruppe, bestehend aus Ni²⁺, Cu²⁺, Zn²⁺, Pd²⁺, Rh²⁺ und Pt²⁺, ausgewählt ist, wenn z 2 ist, und
R^{F} aus der Gruppe, bestehend aus geradkettigen oder verzweigtkettigen, teilweise oder vollständig fluorierten C₁ bis C₁₀-Alkylgruppen, teilweise oder vollständig fluorierten C₆ bis C₂₀-Arylgruppen und teilweise oder vollständig fluorierten C₃ bis C₁₀-Cycloalkylgruppen, ausgewählt ist.

15. Salz mit schwach koordinierendem Anion nach Anspruch 14, dargestellt durch die Formel (3'):
M[(^{F}RO)₃Al-F-Al(OR^{F})₂-F-Al(OR^{F})₃]_{z} (3')
worin z, M und R^{F} wie vorstehend definiert sind.

16. Salz nach Anspruch 14 oder 15, wobei M Ag⁺ oder NBu₄⁺ ist und R^{F} (F₃C)₃C ist.

17. Verwendung eines Salzes nach einem der Ansprüche 14 bis 16 oder hergestellt gemäß dem Verfahren nach einem der Ansprüche 1 bis 13 in ionischen Flüssigkeiten, in Li-Ionen-Batterien, als Leitsalz in der Elektrochemie oder in der homogenen Katalyse.

18. Elementalkoxyverbindung, dargestellt durch die Formel (4):
X(OR^{F})ₘ. (4)
worin X aus der Gruppe, bestehend aus B, Al, Ga, In, P, As und Sb, ausgewählt ist,
m 3 oder 5 ist und
R^{F} aus der Gruppe, bestehend aus geradkettigen oder verzweigtkettigen, teilweise oder vollständig fluorierten C₁ bis C₁₀-Alkylgruppen, teilweise oder vollständig fluorierten C₆ bis C₂₀-Arylgruppen und teilweise oder vollständig fluorierten C₃ bis C₁₀-Cycloalkylgruppen, ausgewählt ist.

19. Elementalkoxyverbindung nach Anspruch 18, worin X Al ist.

## Claims

1. A method for preparing salts of weakly coordinating anions of the type corresponding to the following formula (1), (2) or (3):
M[F-X(OR^{F})ₘ]_{z} (1)
M[(^{F}RO)ₘX-F-X(OR^{F})ₘ]_{z} (2)
M[(^{F}RO)ₘX-F-X(OR^{F})ₙ-F-X (OR^{F})ₘ]_{z} (3)
wherein, in a first step an organyl compound of an element XRₘ is reacted with a partially or completely fluorinated alcohol ^{F}ROH in an organic, aprotic solvent and then, in a second step, the resulting alkoxy compound of the element X(OR^{F})m is reacted with a suitable fluoride salt M_{y}Y_{z} so as to abstract a fluoride ion, if necessary under XFₘ-catalysis, wherein X is selected from the group consisting of B, Al, Ga, In, P, As and Sb,
M is a monovalent or bivalent cation,
m is 3 or 5 and
n is 2, if m is 3, and/or
n is 4, if m is 5,
y is 1 or 2, provided that, if y is 1, Y is a monovalent anion,
or if y is 2, Y is a bivalent anion, and
z is 1 or 2, provided that, if z is 1, M is a monovalent cation,
or if z is 2, M is a bivalent cation.

2. The method according to claim 1 for the production of salts of weakly coordinating anions of the type corresponding to the following formula (1'), (2') or (3'):
M[F-Al (OR^{F})₃]_{z} (1')
M[(^{F}RO)₃Al-F-Al(OR^{F})₃]_{z} (2')
M[(^{F}RO)₃Al-F-Al(OR^{F})₂-F-Al(OR^{F})₃]_{z} (3')
wherein, in a first step an aluminum triorganyl compound AlRₘ is reacted with a partially or completely fluorinated alcohol ^{F}ROH in an organic, aprotic solvent, and then, in a second step, the resulting aluminum trialkoxy compound Al(OR^{F})₃ is reacted with a tetrafluoroborate salt M(BF₄)_{z}, if necessary, under BF₃-catalysis.

3. A method according to claim 1 or 2, wherein the aluminum trialkoxy compound Al(OR^{F})₃ is reacted with the tetrafluoroborate salt M(BF₄)_{z} at a ratio of 1:1, if z is 1, or is reacted at a ratio of 2:1, if z is 2.

4. A method according to claim 1 or 2, wherein the aluminum trialkoxy compound Al(OR^{F})₃ is reacted with the tetrafluoroborate salt M(BF₄)_{z} at a ratio of 2:1, if z is 1, or is reacted at a ratio of 4:1, if z is 2.

5. A method according to any one of the preceding claims, wherein the organic, aprotic solvent is selected from the group consisting of pentane, hexane, heptane, octane, benzene, toluene, cresol, chlorobenzene and trichlorobenzene.

6. A method according to any one of the preceding claims, wherein R is a radical selected from the group consisting of hydrogen, methyl, ethyl, n-propyl, i-propyl, n-butyl, i-butyl, t-butyl, phenyl and tolyl.

7. A method according to any one of the preceding claims, wherein R^{F} is selected from the group consisting of linear or branched, partially or completely fluorinated C₁ to C₁₀ alkyl groups, partially or completely fluorinated C₆ to C₂₀ aryl groups, and partially or completely fluorinated C₃ to C₁₀ cycloalkyl groups.

8. A method according to any one of the preceding claims, wherein, if z is 1, M is selected from the group consisting of alkali metal ions, In⁺, Ti⁺' Ag⁺, Cu⁺, NR'₄⁺, PR'₄⁺, wherein R' is, independently in each case, hydrogen, a linear or branched C₁ to C₂₀-alkyl radical or substituted or unsubstituted aryl radical, and imidazolium, or, if z is 2, M is selected from the group consisting of Ni²⁺, Cu²⁺, Zn²⁺, Pd²⁺, Rh²⁺, and Pt²⁺.

9. A method according to any one of claims 1 to 8, wherein, in a first step, the aluminum triorganyl compound AlMe₃ is reacted with a partially or completely fluorinated alcohol ^{F}ROH in pentane at a ratio of 1:3 and then, in a second step, the resulting aluminum trialkoxy compound Al(OR^{F})₃ is reacted with tetrafluoroborate salt M(BF₄)_{z} at a ratio of 1:1, if z is 1, or at a ratio of 2:1, if z is 2, to yield a compound corresponding to formula (1') above.

10. A method according to any one of claims 1 to 8, wherein, in a first step, the aluminum triorganyl compound AlMe₃ is reacted with a partially or completely fluorinated alcohol ^{F}ROH in pentane at a ratio of 1:3 and, then in a second step, the resulting aluminum trialkoxy compound AI(OR^{F})₃ is reacted with tetrafluoroborate salt M(BF₄)_{z} at a ratio of 2:1, if z is 1, or at a ratio of 4:1, if z is 2, to yield a compound corresponding to formula (2') above.

11. A method according to any one of claims 1 to 8, wherein, in a first step, the aluminum triorganyl compound AlMe₃ is reacted with a partially or completely fluorinated alcohol ^{F}ROH in heptane at a ratio of 1:3 and then, in a second step, the resulting aluminum trialkoxy compound Al(OR^{F})₃ is reacted with tetrafluoroborate salt M(BF₄)_{z} at a ratio of 2:1 if z is 1, or at a ratio of 4:1, if z is 2, to yield a compound corresponding to formula (3') above.

12. A method according to any one of claims 9 to 11, wherein R^{F} is (F₃C)₃C.

13. A method according to any one of claims 9 to 12, wherein M is Ag⁺ or NBu₄⁺.

14. Salts of weakly coordinating anions corresponding to formula (3):
M[(^{F}RO)ₘX-F-X(OR^{F})ₙ,-F-X(OR^{F})ₘ]_{z} (3)
wherein X is selected from the group consisting of B, Al, Ga, In, P, As and Sb,
M is a monovalent or bivalent cation,
m is 3 or 5 and
n is 2, if m is 3, and/or
n is 4, if m is 5,
z is 1 or 2, provided that, if z is 1, M is a monovalent anion,
and/or if z is 2, M is a bivalent anion, and
wherein, if z is 1, M is selected from the group consisting of alkali metal ions, In⁺, Ti⁺' Ag⁺, Cu⁺, NR'₄⁺, PR'₄⁺, wherein R' is, independently in each case, hydrogen, a linear or branched C₁ to C₂₀-alkyl radical or substituted or unsubstituted aryl radical, and imidazolium, or, if z is 2, M is selected from the group consisting of Ni²⁺, Cu²⁺, Zn²⁺, Pd²⁺, Rh²⁺, and Pt²⁺, and R^{F} is selected from the group consisting of linear or branched, partially or completely fluorinated C₁ to C₁₀ alkyl groups, partially or completely fluorinated C₆ to C₂₀ aryl groups, and partially or completely fluorinated C₃ to C₁₀ cycloalkyl groups.

15. The salts of weakly coordinating anions according to claim 14, represented by the formula (3'):
M[(^{F}RO)₃Al-F-Al(OR^{F})₂-F-Al(OR^{F})₃]_{z} (3')
wherein z, M and R^{F} are as defined above.

16. The salts according to claim 14 or 15, wherein M is Ag⁺ or NBu₄⁺ and R^{F} is (F₃C)₃C.

17. Use of a salt according to any one of claims 14 to 16 or prepared according to the method of any one of claims 1 to 13, in ionic fluids, in lithium ion batteries, as a conducting salt in electrochemistry or in homogeneous catalysis.

18. An alkoxy compound of an element, represented by formula (4):
X(OR^{F})ₘ (4)
wherein X is selected from the group consisting of B, Al, Ga, In, P, As and Sb,
m is 3 or 5 and
R^{F} is selected from the group consisting of linear or branched, partially or completely fluorinated C₁ to C₁₀ alkyl groups, partially or completely fluorinated C₆ to C₂₀ aryl groups, and partially or completely fluorinated C₃ to C₁₀ cycloalkyl groups.

19. The alkoxy compound of an element according to claim 18, wherein X is Al.

## Revendications

1. Procédé de production de sels d'anions faiblement coordonnés selon l'une des formules suivantes (1), (2) ou (3) :
M[F-X(OR^{F})ₘ]_{z} (1)
M[(^{F}RO)ₘX-F-X(OR^{F})ₘ]₂ (2)
M[(^{F}RO)ₘX-F-X (OR^{F})ₙ-F-X (OR^{F})ₘ]_{z} (3)
dans lesquelles dans une première étape, un composé organyle élémentaire XRₘ est converti avec un alcool partiellement ou complètement fluoré ^{F}ROH dans un solvant organique, aprotique et ensuite dans une deuxième étape, le composé alcoxy élémentaire obtenu X(OR^{F})ₘ est converti avec un sel de fluorure MyY_{z} pouvant assurer l'abstraction d'un ion fluor éventuellement sous catalyse de XFₘ,
dans lesquelles X est choisi dans le groupe comprenant B, Al, Ga, In, P, As et Sb,
M est un cation monovalent ou bivalent,
m vaut 3 ou 5 et
n vaut 2 si m vaut 3 ou
n vaut 4 si m vaut 5,
y vaut 1 ou 2, à condition que si y vaut 1, Y soit un anion monovalent ou si y vaut 2, Y soit un anion bivalent, et
z vaut 1 ou 2 à condition que si z vaut 1, M soit un cation monovalent ou si z vaut 2, M soit un cation bivalent.

2. Procédé de production de sels d'anions faiblement coordonnés selon l'une quelconque des formules suivantes (1'), (2'), ou (3') selon la revendication 1 :
M[F-Al(OR^{F})₃]₂ (1')
M[(^{F}RO)₃Al-F-X (OR^{F})₃]_{z} (2')
M[(^{F}RO)₃Al-F-Al(OR^{F})₂-F-Al(OR^{F})₃]_{z} (3')
dans lesquelles dans une première étape, un composé tri-organyle d'aluminium AlR₃ est converti avec un alcool partiellement ou complètement fluoré ^{F}ROH dans un solvant organique, aprotique et ensuite dans une deuxième étape, le composé tri-alcoxy d'aluminium obtenu Al (OR^{F})₃ est converti avec un sel de tétrafluoroborate M(BF₄)_{z} éventuellement sous catalyse de BF₃.

3. Procédé selon la revendication 1 ou 2, dans lequel le composé trialcoxy d'aluminium Al(OR^{F})₃ est converti ave le sel de tétrafluoroborate M(BF₄)_{z} en un rapport de 1:1, lorsque z vaut 1 ou en un rapport de 2:1 lorsque z vaut 2.

4. Procédé selon la revendication 1 ou 2, dans lequel le composé trialcoxy d'aluminium Al(OR^{F})₃ est converti avec le sel de tétrafluoroborate M(BF₄)_{z} en un rapport de 2:1, lorsque z vaut 1 ou en un rapport de 4:1 lorsque z vaut 2.

5. Procédé selon l'une quelconque des revendications précédentes, dans lequel le solvant organique aprotique est choisi dans le groupe comprenant le pentane, l'hexane, l'heptane, l'octane, le benzène, le toluène, le crésol, le chlorobenzène et le trichlorobenzène.

6. Procédé selon l'une quelconque des revendications précédentes, dans lequel R est un radical choisi dans le groupe comprenant l'hydrogène, le méthyle, l'éthyle, le n-propyle, le i-propyle, le n-butyle, le i-butyle, le t-butyle, le phényle et le tolyle.

7. Procédé selon l'une quelconque des revendications précédentes, dans lequel R^{F} est choisi dans le groupe comprenant les groupes alkyle en C₁ à C₁₀ à chaîne linéaire ou ramifiée partiellement ou complètement fluorés, les groupes aryle en C₆ à C₂₀ partiellement ou complètement fluorés et les groupes cycloalkyle en C₃ à C₁₀ partiellement ou complètement fluorés.

8. Procédé selon l'une quelconque des revendications précédentes, dans lequel M est choisi dans le groupe comprenant les ions de métal alcalin, In⁺, TI⁺, Ag⁺, Cu⁺, NR'₄⁺, PR'₄⁺, où R' désigne respectivement indépendamment les uns des autres, un atome d'hydrogène, un radical alkyle en C₁ à C₂₀ à chaîne linéaire ou ramifiée ou un radical aryle non substitué ou substitué et l'imidazolium, lorsque z vaut 1 ou M est choisi dans le groupe comprenant Ni²⁺, Cu²⁺, Zn²⁺, Pd²⁺, Rh²⁺ et Pt²⁺ lorsque z vaut 2.

9. Procédé selon l'une quelconque des revendications 1 à 8, dans lequel à la première étape, le composé tri-organyle d'aluminium AlMe₃ est converti en pentane avec l'alcool partiellement ou complètement fluoré ^{F}ROH en un rapport de 1:3 et ensuite dans une deuxième étape, le composé trialcoxy d'aluminium Al(OR^{F})₃ est converti ave un sel de tétrafluoroborate (M(BF₄)_{z} en un rapport de 1: 1 lorsque z vaut 1, ou en un rapport de 2:1 lorsque z vaut 2, en obtenant un composé selon la formule précédente (1').

10. Procédé selon l'une quelconque des revendications 1 à 8, dans lequel à la première étape, le composé tri-organyle d'aluminium AlMe₃ est converti en pentane avec l'alcool partiellement ou complètement fluoré ^{F}ROH en un rapport de 1:3 et ensuite dans une deuxième étape, le composé trialcoxy d'aluminium Al(OR^{F})₃ est converti avec le sel de tétrafluoroborate M(BF₄)_{z} en un rapport de 2:1 lorsque z vaut 1, ou en un rapport de 4:1 lorsque z vaut 2, en obtenant un composé selon la formule précédente (2').

11. Procédé selon l'une quelconque des revendications 1 à 8, dans lequel à la première étape, le composé tri-organyle d'aluminium AlMe₃ est converti en heptane avec l'alcool partiellement ou complètement fluoré ^{F}ROH en un rapport de 1:3 et ensuite dans une deuxième étape, le composé trialcoxy d'aluminium Al(OR^{F})₃ est converti avec un sel de tétrafluoroborate M(BF₄)_{z} en un rapport de 2:1 lorsque z vaut 1, ou en un rapport de 4:1 lorsque z vaut 2, en obtenant un composé selon la formule précédente (3').

12. Procédé selon l'une quelconque des revendications 9 à 11, dans lequel R^{F} est (F₃C)₃C.

13. Procédé selon l'une quelconque des revendications 9 à 12, dans lequel M est Ag₊ ou NBu₄₊.

14. Sel comportant un anion faiblement coordonné, représenté par la formule (3) :
M[(^{F}RO)ₘX-F-X(OR^{F})ₙ-F-X(OR^{F})ₘ]_{z}, (3)
dans laquelle X est choisi dans le groupe comprenant B, Al, Ga, In, P, As et Sb,
M est un cation monovalent ou bivalent,
m vaut 3 ou 5 et
n vaut 2 si m vaut 3 ou
n vaut 4 si m vaut 5,
z vaut 1 ou 2, à condition que si z vaut 1, M soit un cation monovalent ou si z vaut 2, M soit un cation bivalent, et
M est choisi dans le groupe constitué d'ions de métal alcalin, In₊, Tl₊, Ag₊, Cu₊, NR'₄₊, PR'₄₊, où R' est respectivement, indépendamment les uns des autres, un atome d'hydrogène, un radical alkyle en C₁ à C₂₀ à chaîne linéaire ou ramifiée ou un radical aryle non substitué ou substitué et l'imidazolium, lorsque z vaut 1 ou M est choisi dans le groupe comprenant Ni²⁺, Cu²⁺, Zn²⁺, Pd²⁺, Rh²⁺ et Pt²⁺ lorsque z vaut 2 et
R^{F} est choisi dans le groupe comprenant un groupe alkyle en C₁ à C₁₀ à chaîne linéaire ou ramifiée, partiellement ou complètement fluoré, un groupe aryle en C₆ à C₂₀ partiellement ou complètement fluoré et un groupe cycloalkyle en C₃ à C₁₀ partiellement ou complètement fluoré.

15. Sel présentant un anion faiblement coordonné selon la revendication 14, représenté par la formule (3') :
M [(^{F}RO)₃Al-F-Al(OR^{F})₂-F-Al(OR^{F})₃]_{z} (3')
dans laquelle z, M et R^{F} sont tels que définis précédemment.

16. Sel selon la revendication 14 ou 15, dans lequel M est Ag⁺ ou NBu₄⁺ et R^{F} est (F₃C)₃C.

17. Utilisation d'un sel selon l'une quelconque des revendications 14 à 16, ou produit selon le procédé selon l'une quelconque des revendications 1 à 13 dans des liquides ioniques, dans des piles à ions Li, comme sels conducteurs en électrochimie ou dans la catalyse homogène.

18. Composé alcoxy élémentaire représenté par la formule (4):
X(OR^{F})ₘ (4)
dans laquelle X est choisi dans le groupe comprenant B, Al, Ga, In, P, As et Sb,
m vaut 3 ou 5 et
R^{F} est choisi dans le groupe comprenant un groupe alkyle en C₁ à C₁₀ à chaîne linéaire ou ramifiée, partiellement ou complètement fluoré, un groupe aryle en C₆ à C₂₀ partiellement ou complètement fluoré et un groupe cycloalkyle en C₃ à C₁₀ partiellement ou complètement fluoré.

19. Composé alcoxy élémentaire selon la revendication 18, dans lequel X est Al.
